# EUROPÄISCHE PATENTSCHRIFT

(11) **EP 3 704 284 B1**
(45) Veröffentlichungstag und Bekanntmachung des Hinweises auf die Patenterteilung: **22.09.2021**
(21) Anmeldenummer: 18795399.7
(22) Anmeldetag: 22.10.2018
(51) Int. Cl.: C23C 14/06, C23C 28/00, C23C 14/20, C23C 14/34, C23C 14/00

(54) **NANOSTRUKTURIERTE TITAN-MEHRSCHICHTELEKTRODE**
NANOSTRUCTURED TITANIUM MULTILAYER ELECTRODE
ÉLECTRODE MULTICOUCHE EN TITANE NANOSTRUCTURÉE

(30) Priorität: 30.10.2017 DE 102017219425
(43) Veröffentlichungstag der Anmeldung: 09.09.2020
(73) Patentinhaber: Fraunhofer Gesellschaft zur Förderung der angewandten Forschung e.V., 80686 München (DE)
(72) Erfinder: SCHMITZ, Tobias, 97072 Würzburg (DE); SCHWEINLIN, Matthias, 97072 Würzburg (DE); GRÖBER-BECKER, Florian, 97082 Würzburg (DE); HANSMANN, Jan, 97082 Würzburg (DE); METZGER, Marco, 97082 Würzburg (DE); SCHWARZ, Thomas, 97082 Würzburg (DE); WALLES, Heike, 39104 Magdeburg (DE)
(74) Vertreter: Schrell, Andreas
(86) Internationale Anmeldenummer: PCT/EP2018/078934
(87) Internationale Veröffentlichungsnummer: WO 2019/086290

(56) Entgegenhaltungen:
- EP-A1- 2 876 435
- US-A1- 2010 032 842
- A. NORLIN ET AL: "Investigation of interfacial capacitance of Pt, Ti and TiN coated electrodes by electrochemical impedance spectroscopy", BIOMOLECULAR ENGINEERING., Bd. 19, Nr. 2-6, 1. August 2002 (2002-08-01), Seiten 67-71, XP055551979, US ISSN: 1389-0344, DOI: 10.1016/S1389-0344(02)00013-8
- ARYAN N P ET AL: "In vitro study of titanium nitride electrodes for neural stimulation", ENGINEERING IN MEDICINE AND BIOLOGY SOCIETY,EMBC, 2011 ANNUAL INTERNATIONAL CONFERENCE OF THE IEEE, IEEE, 30. August 2011 (2011-08-30), Seiten 2866-2869, XP032319361, DOI: 10.1109/IEMBS.2011.6090791 ISBN: 978-1-4244-4121-1

## Beschreibung

Die vorliegende Erfindung betrifft eine mehrschichtige Elektrode aus Titan und Titannitrid, geeignet für die Aufbringung auf thermoplastische Substrate, besonders zum Zwecke der Impedanzmessung in wässrigen biologischen Medien, sowie Verfahren zu deren Herstellung.

Die Erfindung betrifft das technische Gebiet der Bestimmung elektrischer Parameter von biologischen Zellen und Geweben in flüssigen biologischen Medien, besonders Mittel zur elektrischen Impedanzmessung des komplexen elektrischen Widerstandes, beispielsweise des transepithelialen Widerstandes (TEER) von kultivierten Zellen oder ein- oder mehrschichtigen Geweben in Zellkulturkammern oder Reaktormodulen.

Durch kultivierte biologische Zellen können *in vitro* Gewebe oder Organmodelle hergestellt werden, woran beispielsweise die Wirkung von Substanzen, besonders von pharmazeutischen Wirkstoffen oder Inhaltsstoffen von Kosmetika oder Lebensmitteln, auf ihre biologische Verträglichkeit hin untersucht werden. Besonders kann so die Durchführung von ansonsten üblichen Tierversuchen vermieden werden. Ein Verfahren zur laufenden und damit zerstörungsfreien Untersuchung von physiologischen Parametern solcher *in vitro* Modelle ist die Impedanzspektroskopie. Anhand der komplexen technischen Widerstände der untersuchten *in vitro* Gewebe kann auf die Integrität der Gewebe, beispielsweise auf die Schichtung und Intaktheit, aber auch auf physiologische Vorgänge an oder in den Geweben, beispielsweise Transportvorgängen oder Kanalöffnungen, rückgeschlossen werden. Vorteilhafterweise werden solche *in vitro* Gewebe in parallelen Ansätzen in Mehrfach-Kulturplatten (Multiwellplatten) durchgeführt. Idealerweise werden diese parallelen Ansätze automatisiert in Kultivierungsautomaten verarbeitet. Die verwendeten Zellkulturplatten sind daher in der Regel Einweggefäße. Einer stärkeren Nutzung der Impedanzspektroskopie steht diese Art der parallelen Ansätze jedoch entgegen, da für die Impedanzspektroskopie momentan Messelektroden durchgeführt werden, die auf Basis teurer Edelmetalle wie Gold oder Platin aufgebaut sind. Diese waren bisher erforderlich, da sie eine ausreichende chemische Stabilität besitzen und eine lange und zuverlässige Verbindung als Elektrode mit gleichbleibenden elektrischen Eigenschaften garantieren. Solche Materialien sind für automatisierbare *in vitro* System aufgrund der hohen Kosten nur bedingt geeignet.

Gleichzeitig hat sich gezeigt, dass gerade die Edelmetall-basierten Elektroden oder davon abgeleitete Elektrodenmaterialien gerade im niedrigen Frequenzbereich ungünstige Impedanzeigenschaften wie hohen Widerstand und hohe Phasenverschiebung aufweisen.

Infolge der hohen Anschaffungskosten dieser teuren Elektrodenmaterialien, sind darauf basierte Inpedanzspektroskopie-Systeme zur mehrfachen Benutzung ausgelegt. Nach jeder Benutzung müssen sie gründlich gereinigt und sterilisiert werden, um das Risiko einer Kontamination gering zu halten. Dies ist in automatisierbaren *in vitro* Systemen nicht zweckmäßig.

Bisher wurden daher in automatisierbare *in vitro* Systemen solche Impedanzmessungen nur seriell oder in parallelen Ansätzen geringer Zahl eingesetzt, was zum einen die Aussagekraft der Impedanzmessung reduziert.

Die derzeit verfügbaren Messkammern sind daher zum einen mit hohen Anschaffungs- und Betriebskosten verbunden, gleichzeitig besitzen die in diesen verfügbaren Messkammern verwendeten Elektrodenmaterialien eine vergleichsweise geringe Sensitivität. Die Notwendigkeit der Sterilisierbarkeit bei der wiederkehrenden Verwendung verlangt außerdem, dass die Messkammern aus vergleichsweise teuren Polymeren, die autoklavierbar sein müssen, hergestellt werden. Relevanter Stand der Technik ist in A. Norlin ET AL: "Investigation of interfacial capacitance of Pt, Ti and TiN coated electrodes by electrochemical impedance spectroscopy",BIOMOLECULAR ENGINEERING., Bd. 19, Nr. 2-6, 1. August 2002 (2002-08-01), Seiten 67-71, DOI: 10.1016/S1389-0344(02)00013-8, offenbart.

Das der vorliegenden Erfindung zugrunde liegende technische Problem bestand demgemäß darin, eine kostengünstige Elektrode, geeignet für die Impedanzmessung in biologischen Medien, bereitzustellen, welche die vorbeschriebenen Nachteile überwindet und insbesondere kostengünstig herstellbar ist, verbesserte elektrische Eigenschaften bei der Impedanzmessung aufweist und/oder auf gängige Standardzellkulturplatten aus niedrigschmelzenden Thermoplasten oder anderen Mitteln für die Zell- und Gewebekultur aufbringbar ist.

Das technische Problem wird vollständig gelöst durch die Bereitstellung einer neuartigen Titan-basierten Elektrode, welche mehrschichtig ist, und insbesondere zwei übereinanderliegende Titannitridschichten aufweist, wobei eine titanreiche Titannitrid-Zwischenschicht mit stöchiometrisch erhöhtem Titananteil und eine titanärmere Titannitrid-Deckschicht mit stöchiometrisch vermindertem Titananteil auf einer Titan-Trägerschicht ausgebildet sind.

Ein erster Gegenstand der vorliegenden Erfindung ist daher eine Titanelektrode gemäß Anspruch 1, welche besonders für die Impedanzmessung in wässrigen biologischen Medien geeignet ist und insbesondere auf ein thermoplastisches polymeres Substrat ohne weiteres aufbringbar ist, wobei die Titanelektrode auf einem Substrat zuerst eine Titan-Trägerschicht aus elementarem Titan, eine titanreiche Titannitrid-Zwischenschicht, welche auf der Titan-Trägerschicht aufliegt, und eine titanärmere Titannitrid-Deckschicht, welche auf der titanreichen Titannitrid-Zwischenschicht aufliegt, gebildet ist.

Spezifisch ist das:
- eine (untere) Ti-Trägerschicht, bestehend aus elementarem Titan (Ti),
- mindestens eine titanreiche Titannitrid (TiNₓ)-Zwischenschicht, enthaltend oder vollständig bestehend aus TiN*ₓ*, wobei *x* kleiner ist als 1 oder x gleich 1 ist, welche, insbesondere unmittelbar und direkt, auf der Ti-Trägerschicht aufliegt, und
- eine titanarme Titannitrid (TiN_{y})-Deckschicht, enthaltend oder vollständig bestehend aus TiN*_{y}*, wobei *y* größer ist als 1, welche, insbesondere unmittelbar und direkt, auf der vorgenannten titanreichen TiN*ₓ*-Zwischenschicht aufliegt.

Bevorzugt ist in der oben genannten erfindungsgemäßen Schichtenfolge der Titanelektrode genau eine titanreiche Titannitrid (TiN*ₓ*)-Zwischenschicht vorgesehen. In bevorzugter Ausgestaltung besteht die einzige TiN*ₓ*-Zwischenschicht aus TiN*ₓ*, mit x von 0,3 bis 1,0, besonders bevorzugt von 0,6 bis 0,9.

In einer alternativen bevorzugten Variante sind in dieser Schichtenfolge mehr als eine titanreiche Titannitrid (TiN*ₓ*)-Zwischenschicht vorgesehen, wobei eine erste untere titanreiche Zwischenschicht einen höheren Titangehalt als die jeweils darauf aufliegende weitere titanreiche Zwischenschicht aufweist. Die unterste TiN-Schicht der Elektrode ist die titanreichste. Die in der Schichtenfolge nachfolgenden TiN-Schichten sind jeweils weniger titanreich, beziehungsweise titanärmer. Eine erste, titanreichste TiN*ₓ*-Zwischenschicht weist dabei bevorzugt *x* von 0,3 bis 0,6, besonders bevorzugt von 0,3 bis 0,5, auf, eine unmittelbar darauf aufliegende weitere titanreiche TiN*ₓ*-Zwischenschicht weist dabei bevorzugt *x* von 0,5 bis 1,0 besonders bevorzugt von 0,6 bis 0,9 auf, wobei die weitere Zwischenschicht einen geringeren Titangehalt aufweist, als die unmittelbar darunter liegende Zwischenschicht.

In bevorzugter Ausgestaltung besteht die TiN*_{y}*-Deckschicht aus TiN*_{y}*, mit y von 1,1 bis 1,5, besonders bevorzugt von 1,2 bis 1,4.

Die Erfinder fanden überraschend, dass eine solche mehrschichtige Elektrode, die mittels physikalischer Gasphasenabscheidung (PVD), spezifisch durch reaktive Kathodenzerstäubung (reaktives Sputtern) ohne weiteres auch auf niedrigschmelzenden thermoplastischen Substraten vorzugsweise in einem einzigen Arbeitsschritt aufgebracht werden kann. Bevorzugt sind also vergleichsweise dünne Elektrodenschichten. Es hat sich gezeigt, dass dünne Elektrodenschichten ohne oder ohne nennenswerte thermische Belastung des Substratmaterials hergestellt werden können. Hierbei steht vor allem im Vordergrund, die Erweichung des polymeren Substrats, besonders bei niedrigschmelzenden Kunststoffen, zu vermeiden. Ein Überschreiten der Glasübergangstemperatur bei amorphen Thermoplasten beziehungsweise der Schmelztemperatur bei teilkristallinen Thermoplasten soll verhindert werden oder zumindest auf den Ort der Abscheidung der Elektrodenschichten begrenzt bleiben.

Bevorzugt beträgt die Gesamtdicke der mehrschichtigen Elektrode 3000 nm oder weniger, insbesondere weniger als 2000 nm. Bevorzugt beträgt die Dicke der TiN-Schichten der mehrschichtigen Elektrode 3000 nm oder weniger oder 2000 nm oder weniger, insbesondere aber weniger als 1000 nm. Bevorzugt aber weist dabei zumindest die mindestens eine TiN*ₓ*-Zwischenschicht eine Dicke von 100 bis 500 nm, bevorzugt von 150 bis 250 nm, auf. In Falle einer Elektrode mit mehr als einer Zwischenschicht, kann jede der Zwischenschichten bevorzugt eine Dicke von 100 bis 500 nm, besonders bevorzugt von 150 bis 250 nm, aufweisen. Bevorzugt weist die TiN*_{y}*-Deckschicht eine Dicke von 500 bis 5000 nm, besonders bevorzugt von 300 bis 2000 nm, auf.

Die erfindungsgemäße dreischichtige Elektrode weißt überraschenderweise bereits bei dünnen Schichten hervorragende elektrische Eigenschaften auf, besonders in Bezug auf das Impedanzverhalten, vor allem in dem für die impedanzspektroskopische Messung von biologischem Material wesentlichen Frequenzbereich. Die TEER-Messung findet gemäß anerkannter Standards bei etwa 12,5 Hz statt. Die erfindungsgemäße zumindest dreischichtige Titanelektrode ist besonders gut geeignet für die impedanzspektroskopische Messung biologischer Zellen und Geweben in wässrigen biologischen Medien, insbesondere in an sich bekannten Zellkulturmedien. Im Gegensatz zu den bekanntermaßen dazu eingesetzten Edelmetallelektroden, welche auf Gold und/oder Platin basieren, weist die erfindungsgemäße Titanelektrode gerade im niedrigen Frequenzbereich, welcher für die Charakterisierung von *in vitro* Geweben besonders wichtig ist, eine deutlich verbesserte Sensitivität auf. Auch im Gegensatz zu anderen dünnen Titanelektroden, die eine Ti-Basisschicht und nur eine einzige darauf abgeschiedene Titannitrid besitzen, ist die erfindungsgemäße Titanelektrode besonders in diesem niedrigen Frequenzbereich ebenfalls elektrisch deutlich überlegen. Es hat sich gezeigt, dass die titanreiche TiN*ₓ*-Zwischenschicht eine höhere Dichte aufweist und daher eine höhere Leitfähigkeit aufweist und dass die titanärmere TiN*_{y}*-Deckschicht eine geringere Dichte aufweist und besonders aufgrund ihrer offeneren Struktur besonders gute Impedanzeigenschaften besitzt; die titanärmere TiN*_{y}*-Deckschicht besitzt eine besonders niedrige Impedanz. Besonders aus dem Zusammenspiel von leitfähigerer Zwischenschicht und niedrigimpedanter Deckschicht lassen sich die überraschend guten elektrischen Eigenschaften begründen.

Es hat sich gezeigt, dass TiN*_{y}*-Deckschicht und TiN*ₓ*-Zwischenschicht am Besten in einem bestimmten Dickenverhältnis zueinander vorhanden sind. Bevorzugt beträgt, insbesondere im Falle einer dreischichtigen Titanelektrode mit einer Deckschicht und mit einer einzigen Zwischenschicht, die Dicke der TiN*_{y}*-Deckschicht das 1- bis 10-Fache, besonders bevorzugt das 1,5- bis 4-Fache, der Dicke der TiN*ₓ*-Zwischenschicht. Besonders ist dabei bevorzugt vorgesehen, dass die drei Schichten, welche die Elektrode bilden, zueinander in einem Dickenverhältnis von 1-5 / 1 / 1-10, bevorzugt 1-5 / 1 / 1,5-4, bezogen auf Ti-Trägerschicht / TiN*ₓ*-Zwischenschicht / TiN*_{y}*-Deckschicht, stehen.

Bevorzugt beträgt, insbesondere im Falle einer Titanelektrode mit mehr als drei Schichten, das heißt mit einer Deckschicht und mit mehr als einer Zwischenschicht, die Dicke der TiN*_{y}*-Deckschicht das 0,5- bis 10-Fache, besonders bevorzugt das 0,5-bis 4-Fache, der Dicke der TiN*ₓ*-Zwischenschichten. Besonders ist dabei bevorzugt vorgesehen, dass die Schichten, welche die Elektrode bilden, zueinander in einem Dickenverhältnis von 1-5 / 1 / 0,5-10, bevorzugt 1-5 / 1 / 0,5-4 , bezogen auf Ti-Trägerschicht / Gesamtheit der TiN*ₓ*-Zwischenschichten / TiN*_{y}*-Deckschicht, stehen.

Gleichzeitig zeigt sich, dass die erfindungsgemäße Titanelektrode eine hohe chemische Stabilität aufweist und daher ohne weiteres für die laufende und längerfristige Impedanzmessung während der Kultivierung oder Bestimmung von Wirkstoffeffekten bei Reaktoren für Zellen oder *in vitro* Geweben verwendet werden kann.

Aufgrund der niedrigen Prozesstemperatur bei der Erstellung der erfindungsgemäßen Titanelektrode können besonders auch handelsübliche standardisierte Einweg-Zellkulturplatten, die regelmäßig aus niedrigschmelzenden Thermoplasten, beispielsweise im Spritzguss oder vergleichbaren Verfahren, hergestellt sind, direkt beschichtet werden, sodass erfindungsgemäße Titanelektroden vorteilhafterweise unmittelbar direkt auf Einweg-Zellkulturplatten oder ähnliche Systeme zur Aufbewahrung oder zur Leitung von wässrigen Zellkulturmedien, aufgebracht werden können. Auf diese Weise können Einweg-Messkammern mit Titanelektroden günstig und in großer Zahl bereitgestellt werden, was es ermöglicht, in der automatisierbaren *in vitro* Kultivierung parallele Impedanzmessungen in sämtlichen Zellkulturgefäßen gleichzeitig und gegebenenfalls über die gesamte Dauer der Kultivierung der Gewebe und/oder der Substanzeinwirkung laufend durchzuführen. Die Notwendigkeit des Einsatzes von Mehrwegsystemen und deren aufwändige Reinigung besteht nicht mehr.

Ein weiterer Gegenstand der Erfindung ist ein neuartiges Verfahren zur Herstellung einer erfindungsgemäßen Titanelektrode gemäß Anspruch 10, besonders zum Aufbringen der Titanelektrode auf eine Zellkulturplatte oder allgemein ein Messmodul aus einem polymeren thermisch empfindlichen Werkstoff, welcher als Substrat für die Titanelektrode dient.

Das erfindungsgemäße Verfahren basiert auf der physikalischen Gasphasenabscheidung in Form der reaktiven Kathodenzerstäubung und findet in Gegenwart eines Prozessgases statt. Das Prozessgas setzt sich, je nachdem ob eine reine Titanschicht oder eine Titannitridschicht abgeschieden werden soll, aus einem Inertgas, vorzugsweise Argon, beziehungsweise aus einem Gemisch aus dem Inertgas, vorzugsweise Argon, und Stickstoffgas zusammen.

Erfindungsgemäß enthält das Verfahren zumindest die folgenden Schritte: Schritt (b): Titan wird auf ein Substrat in Gegenwart eines Prozessgases, enthaltend Argon und Stickstoff, gesputtert, und zwar bei einer erhöhten Generatorleistung, um eine erste Schicht bestehend aus titanreichem Titannitrid auf einer Titan-Trägerschicht zu deponieren. Anschließend wird in Schritt (c) die Generatorleistung unmittelbar reduziert und dann in Schritt (d) Titan auf das Substrat bei verminderter Generatorleistung gesputtert, um eine zweite Schicht, bestehend aus titanarmem Titannitrid auf der gebildeten titanreichen Titannitridschicht zu deponieren.

In einer Variante der Erfindung wird eine Titanelektrode ausgebildet, die mehr als eine titanreiche Zwischenschicht in ihrer Schichtenfolge aufweist. Dazu ist besonders vorgesehen, dass die Schritte b) und c) mehrfach durchlaufen werden, um mindestens eine weitere Titannitrid-Zwischenschicht, bestehend aus dem titanreichem TiN*ₓ*, auf einer ersten Titannitrid-Zwischenschicht zu deponieren, wobei die weitere Titannitrid-Zwischenschicht titanärmer ist als die jeweils zuvor abgeschiedene titanreichere Titannitrid-Zwischenschicht. Dazu ist erfindungsgemäß besonders vorgesehen, dass in den jeweiligen Schritten c) bei jedem Durchlauf der Schritte b) und c) die Generatorleistung jeweils gegenüber dem vorhergehenden Durchlauf reduziert wird, um die titanärmere weitere Titannitrid-zwischenschicht auf einer der jeweils zuvor abgeschiedenen titanreicheren Titannitrid-Zwischenschicht (30) zu bilden.

Erfindungsgemäß ist besonders vorgesehen, dass die Schritte b) bis d) in einem Durchgang unmittelbar aufeinanderfolgend durchgeführt werden, um die beiden Titannitridschichten zu bilden. Bevorzugt ist vorgesehen, dass die erhöhte Generatorleistung in Schritt b) 1,2- bis 2,4-fach so hoch ist wie die Generatorleistung in Schritt d). Um eine Elektrode mit mehreren Zwischenschichten zu bilden, ist beispielsweise vorgesehen, dass in Schritt b) die erste Zwischenschicht bei 2,4-fach erhöhter Generatorleistung deponiert wird, um eine besonders titanreiche Titannitrid-Zwischenschicht zu erzeugen, die Generatorleistung in Schritt c) unmittelbar reduziert wird, damit in Schritt b) des weiteren Durchlaufs eine zweite (oder weitere) Zwischenschicht bei nur 1,2-fach erhöhter Generatorleistung deponiert wird, um eine weniger titanreiche weitere Titannitrid-Zwischenschicht zu erzeugen, und anschließend die Generatorleistung in Schritt c) des weiteren Durchlaufs erneut unmittelbar reduziert wird, um schließlich in Schritt d) bei nicht erhöhter Generatorleistung die titanarme Titannitrid-Deckschicht zu erzeugen.

Bevorzugt ist vorgesehen, dass während der Schritte b) bis d) die Zusammensetzung des Prozessgases (Argon/Stickstoff) besonders bezogen auf den Stickstoff-Partialdruck, konstant gehalten wird.

Vorzugsweise geht Schritt b) noch der Schritt a) voraus, worin eine Titan-Trägerschicht auf einem Substrat, vorzugsweise auf einem polymeren Substrat besonders bevorzugt aus einem thermoplastischen Substrat deponiert wird. Vorzugsweise findet Schritt a) durch Sputtern von Titan auf das polymere Substrat in Gegenwart eines Prozessgases, bestehend aus Inertgas, bevorzugt Argon, in einem PVD-Verfahren der reaktiven Kathodenzerstäubung statt.

Das erfindungsgemäße Verfahren ist also besonders dadurch gekennzeichnet, dass die beiden sich in ihrem Titangehalt unterscheidenden Titannitridschichten unmittelbar aufeinanderfolgend abgeschieden werden, wobei das stöchiometrische Verhältnis in dem Titannitrid durch unmittelbares Verändern der Generatorleistung bei der Kathodenzerstäubung erzeugt wird. Eine erhöhte Generatorleistung erzeugt dabei eine titanreiche Titannitridschicht, eine dazu vergleichsweise verminderte Generatorleistung erzeugt dabei eine titanärmere Titannitridschicht. Durch dieses unmittelbare Umschalten der Generatorleistung während des Abscheideprozesses ist es vorteilhafterweise möglich, die Prozessdauer der Schichtabscheidung auf dem Substrat insgesamt zu reduzieren, was verhindert, dass sich das Substrat übermäßig erwärmt. Dies erlaubt vorteilhafterweise die Verwendung von niedrigschmelzenden Thermoplasten als Substrat, wodurch es ermöglich wird, normale Standardzellkulturplatten oder andere polymere Laborgefäße zur Aufbewahrung und Führung flüssiger biologischer Medien mit einer Titanelektrode zu versehen, die besonders geeignet ist für die Impedanzmessung in diesen biologischen Medien. Auf diese Weise lassen sich an herkömmlichen Zellkulturgefäßen und ähnlichen Laborgeräten einfach und kostengünstig hochwertige Elektroden für die Impedanzmessung ausbilden. Das erfindungsgemäße Verfahren ist daher insbesondere derart ausgestaltet, dass das Substrat (10) aus einem niedrigschmelzenden Thermoplast besteht oder dieses enthält.

Das polymere Substrat ist bevorzugt ausgewählt aus der Gruppe der, insbesondere niedrigschmelzenden, Thermoplaste, bevorzugt bestehend aus: Polystyrol (PS), Polyamid (PA), besonders PA6, PA6.6; Acrylnitril-Butadien-Styrol (ABS); Polylactat (PLA); Polycarbonat (PC); Polyethylenterephthalat (PET), Polyethylen (PE), Polycaprolacton (PCT); Polypropylen (PP); Polyetheretherketon (PEEK), Polyimid (PI), Polyurethan (PU) und Polyvinylchlorid (PVC) sowie Kombinationen, Mischungen und Co-Polymeren davon.

Demgemäß können Standardzellkulturgefäße wie Multiwellplatten, aber auch Membranreaktoren, Hohlfasermodule, aber auch andere Laborgeräte zur Aufbewahrung und Führung von flüssigen biologischen Medien, wie beispielsweise Schlauchanschlussstücke, Fittings, Schlauchstücke etc. unmittelbar und direkt mit dieser Titanelektrode versehen werden.

Die Erfindung schließt in einer Variante jedoch nicht aus, dass als Substrat auch andere, thermisch stabile Werkstoffe eingesetzt werden können. Der mit der erfindungsgemäßen Titanelektrode erreichte technische Vorteil erstreckt sich nicht nur auf die verbesserte Anwendbarkeit auf polymeren Werkstoffen. Die hervorragenden elektrischen Eigenschaften der erfindungsgemäßen Titanelektrode, insbesondere bei geringen Schichtdicken, werden auch erreicht, wenn thermisch stabile Werkstoffe eingesetzt werden. Diese sind bevorzugt ausgewählt aus der Gruppe der Metalle und Metalloxide, besonders bestehend aus: Titan, Edelstahl, Magnesium, Aluminiumoxide, Titanoxid und Kombinationen davon, und zwar bevorzugt in massiver Form oder als insbesondere offenporige Sinterstruktur. Beispiele sind Substrate aus gesinterten Keramiken.

Gegenstand der Erfindung ist daher auch ein Verfahren zur Herstellung eines Messmoduls, worin das flüssige Medium geführt werden kann, für die Impedanzmessung in wässrigen biologischen Medien, enthaltend die Schritte: Bereitstellen des Moduls als Substrat der Elektrode und Abscheiden der Titanelektrode auf dem Substrat mittels des hierin beschriebenen erfindungsgemäßen Verfahrens.

Gegenstand der Erfindung ist daher auch ein (allgemeines) Messmodul, worin das flüssige Medium geführt werden kann, für die Impedanzmessung in wässrigen biologischen Medien, enthaltend oder bestehend aus einem Substrat und mindestens einer bevorzugt unmittelbar auf diesem Substrat abgeschiedene mehrschichtige Titanelektrode wie sie hierin beschrieben ist.

Ein solches Messmodul, woran die erfindungsgemäße Titanelektrode ausgebildet sein kann, ist bevorzugt ausgewählt aus: Zellkulturgefäßen, Multiwellplatten, Membranreaktoren, Hohlfasermodulen, Schlauchanschlussstücken.

Eine besonders bevorzugte Ausgestaltung ist eine an sich bekannte Multiwellplatte (Mikrotiterplatte), die zumindest im Bodenbereich eines, insbesondere jedes, Wells zumindest eine erfindungsgemäße Titanelektrode trägt. Diese ist bevorzugt direkt auf dem Bodenabschnitt der polymeren Multiwellplatte abgeschieden. Bevorzugt sind elektrisch leitende Strukturen oder Leiterbahnen zur Kontaktierung der Elektrode an dem Well ausgebildet. Diese enden beispielsweise an der Oberseite des Wells oder laufen bevorzugt zu einer gemeinsamen integralen Kontaktierungsfläche am Rand der Multiwellplatte, um eine bevorzugt mehrkanalige Kontaktierung aller Elektroden in allen Wells zu ermöglichen. Dies ist besonders für eine automatisierte Bearbeitung, das heißt automatisiertes Handling der Kulturplatten zweckmäßig. Die Leiterbahnen zur Kontaktierung der Elektrode sind bevorzugt ebenfalls in der Art und Dimensionierung der Elektrode ausgebildet. Besonders bevorzugt werden Elektrode und Leiterbahn in einem Bearbeitungsschritt, das heißt besonders mit dem hierin beschriebenen Beschichtungsverfahren, auf das Substrat aufgetragen. Demgemäß ist bevorzugt, dass die gesamte Stromleitung über den dünnen Querschnitt des Schichtsystems führt, wobei der Widerstand hier vergleichbar gering ist, wie bei einer Schicht die auf einem leitenden Vollmaterial aufgebracht wäre.

Eine andere bevorzugte Ausgestaltung ist ein Schlauchanschlussstück (Fitting) oder Zwischenstück an oder zur Benutzung an einem Reaktormodul. Dieses Anschlussstück dient zweckmäßigerweise zur Durchleitung von biologischen Medien von und aus einem Reaktormodul. Das Anschlussstück ist als Messmodul ausgebildet und weist dazu zumindest an der zu dem biologischen Medium hin gewandten Seite (Lumen) die erfindungsgemäße Titanelektrode auf. Die ist bevorzugt zumindest dort direkt auf dem polymeren Werkstoff des Anschlussstücks selbst abgeschieden. Leitfähige Strukturen, bevorzugt die Titanelektrodenbeschichtung selbst, führen von der Medienkontaktseite (medienführendes Lumen) nach Außen, wo an sich bekannte Mittel zur elektrischen Kontaktierung angreifen können. In einer einfach aufzubauenden Variante sind die Anschlussstücke laborübliche Luer-Lock-Schlauchanschlussstücke zum Anschluss von medienführenden Schläuchen an entsprechende Luer-Lock Klemmfittings. Diese Schlauchanschlussstücke aus polymerem Material selbst sind mit der Titanelektrodenbeschichtung versehen.

In einer anderen erfindungsgemäßen Ausführung des Anschlussstücks, das heißt besonders Fitting oder auch Schlauch, ist in dieses eine separate Trägerstruktur eingesetzt, worauf die erfindungsgemäße Titanelektrode abgeschieden ist. Diese Trägerstruktur ist bevorzugt ein Netz oder Gitter aus metallischem Werkstoff, insbesondere Edelstahl, oder besonders bevorzugt aus einem vorgenannten polymeren Material, insbesondere Nylon (PA6, PA6.6). Bei einer einfach aufbaubaren Variante davon ist die netz- oder gitterförmige Titanelektrode der Erfindung direkt in einen Polymerschlauch für flüssige Medien, insbesondere einen laborüblichen Silikonschlauch eingesetzt.

Eine Variante davon ist ein Hohlmembranreaktor, der im Bereich der Schlauchanschlüsse. besonders an den Zu- und Abläufen, die erfindungsgemäße Titanelektrode aufweist. Die Titanelektrodenbeschichtung ist erfindungsgemäß bevorzugt an der Innenseite der Schlauchanschlüsse ausgebildet, wo diese mit den flüssigen Medien wie Dialysaten in Kontakt stehen. Alternativ ist mindestens ein Teil des Reaktorgehäuses, besonders im Bereich der Endkappen eines Hohlmembranreaktors mit einer erfindungsgemäßen Titanelektrodenbeschichtung versehen.

Die Erfindung wird durch die nachfolgenden Figuren und Beispiele erläutert, ohne dass diese beschränkend zu verstehen wären.

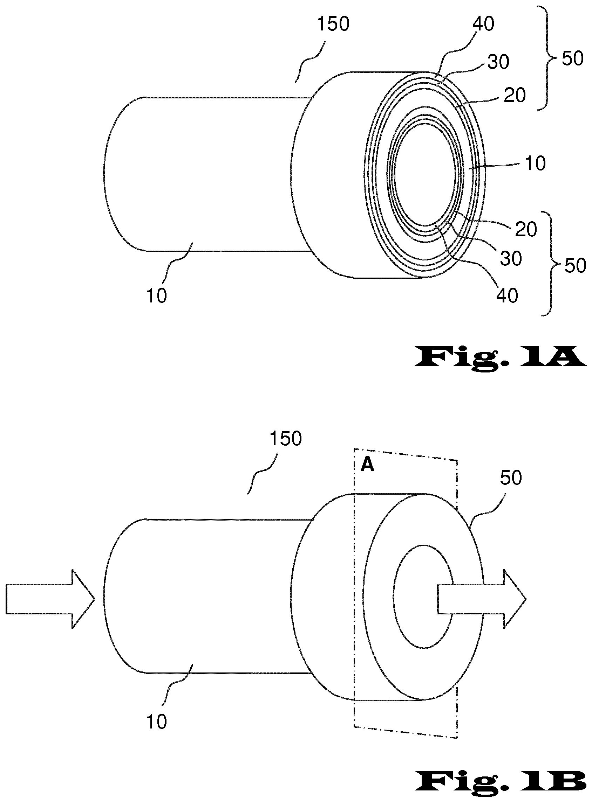

Figuren 1A und 1B zeigen eine schematische Ansicht eines erfindungsgemäßen Messmoduls in Form eines Anschlussstücks (150). Die Darstellung ist nicht skaliert. Auf einem im Prinzip röhrenförmigen Träger aus Polymer als Substrat (10) ist zumindest an einem Abschnitt eine erfindungsgemäße mehrschichtige Titanelektrode (50) ausgebildet. Diese besitzt, wie in der Schnittansicht in Figur 1A gezeigt (Schnittebene "A" wie in Figur 1B), einen jeweils dreischichtigen Aufbau: eine Ti-Schicht (20) ist direkt auf dem Substrat (10) abgeschieden, darauf eine titanreiche TiN-Zwischenschicht (30), darauf eine titanarme TiN-Deckschicht (40). Die Schrägansicht in Figur 1B zeigt das Anschlussstück (150) zur Leitung von flüssigem Medium (Pfeil) mit der Titanelektrode (50), die sich ins medienführende Innere (Lumen) des rohrförmigen Trägers (10) und, zum Zwecke der elektrischen Kontaktierung, in der dargestellten bevorzugten Variante auch auf die Außenseite hin erstreckt.

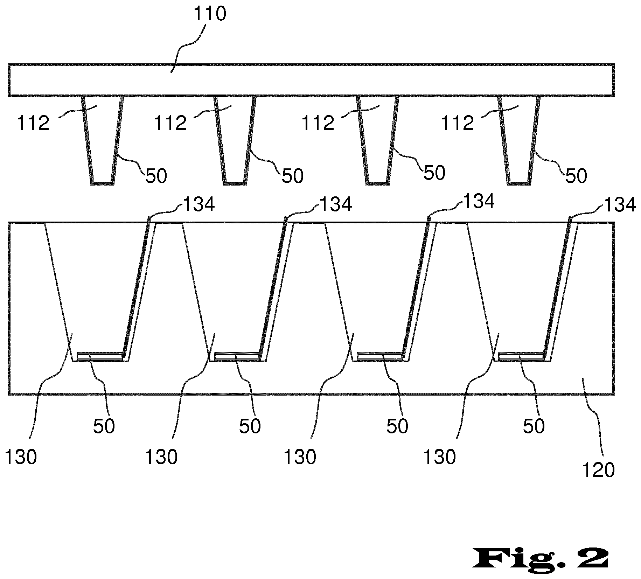

Figur 2 zeigt einen schematischen Querschnitt durch eine erfindungsgemäße Messanordnung mit Zellkulturplatte (Multiwellplatte) mit mehreren nebeneinander auf einen gemeinsamen Träger (120) angeordneten Vertiefungen (Wells) (130). Auf dem Boden jeder Vertiefung (130) ist direkt eine erfindungsgemäße mehrschichtige Titanelektrode (50) ausgebildet. In der dargestellten Ausführung führt eine elektrische Leiterbahn (134) von der Elektrode (50) zumindest bis zu dem oberen Rand der Vertiefung (130) und ermöglicht die elektrische Kontaktierung. Figur 2 zeigt zusätzlich einen bevorzugt einsetzbaren Deckel oder Stempel (110). Dieser besitzt Elektrodenträger (112), die bei Gebrauch jeweils in die Vertiefungen (130) der Multiwellplatte abgesenkt werden können. An den Elektrodenträgern (112) ist jeweils eine erfindungsgemäße mehrschichtige Titanelektrode (50) ausgebildet. Diese bildet im Gebrauch die elektrische Gegenelektrode zu der Elektrode am Boden der Vertiefung (130) der Multiwellplatte.

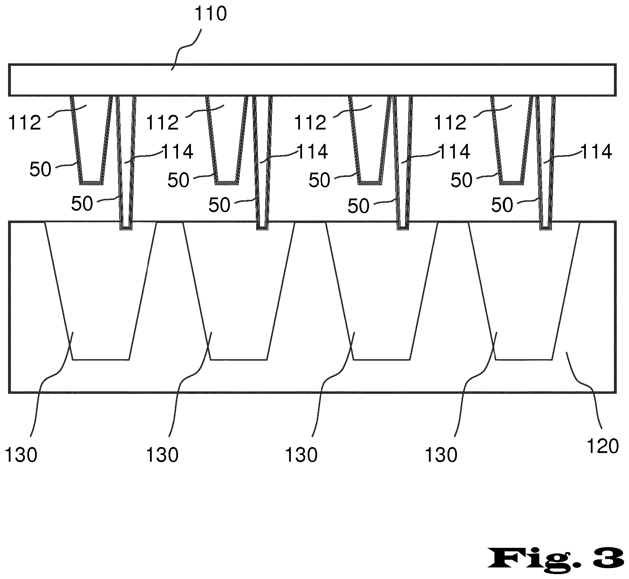

Figur 3 zeigt einen schematischen Querschnitt durch eine andere erfindungsgemäße Messanordnung zur Verwendung mit einer herkömmlichen Zellkulturplatte (Multiwellplatte) mit mehreren nebeneinander auf einen gemeinsamen Träger (120) angeordneten Vertiefungen (Wells) (130). Die erfindungsgemäße Messanordnung ist als Deckel oder Stempel (110) ausgebildet. Dieser besitzt obere Elektrodenträger (112) und untere Elektrodenträger (114), die bei Gebrauch jeweils in die Vertiefungen (130) der Multiwellplatte abgesenkt werden können. An den Elektrodenträgern (112, 114) ist jeweils eine erfindungsgemäße mehrschichtige Titanelektrode (50) ausgebildet. Oberer Elektrodenträger (112) und unterer Elektrodenträger (114) dienen im Gebrauch als Elektrode und Gegenelektrode in der jeweiligen Vertiefung (130).

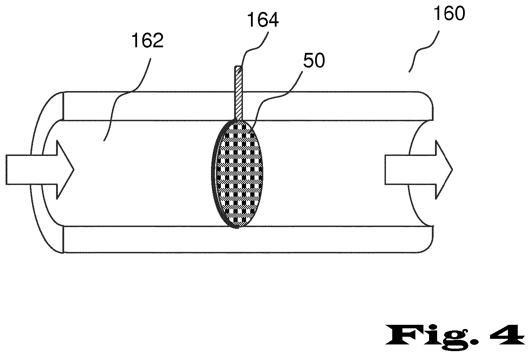

Figur 4 zeigt eine schematische perspektivische Teilschnitt-Ansicht eines erfindungsgemäßen Messmoduls in Form eines Anschlussstücks (160) zur Leitung von flüssigem Medium (Pfeile). Die Darstellung ist nicht skaliert. In dem medienführenden Lumen (162) des Anschlussstücks (160) ist ein im Prinzip netz- oder gitterförmiger Träger aus Polymer eingesetzt, der als Substrat für die erfindungsgemäße mehrschichtige Titanelektrode (50) dient. Ein Kontaktierungselement (164) dient dem elektrischen Anschluss. Das Kontaktierungselement (164) kann durch den beschichteten Träger selbst gebildet sein.

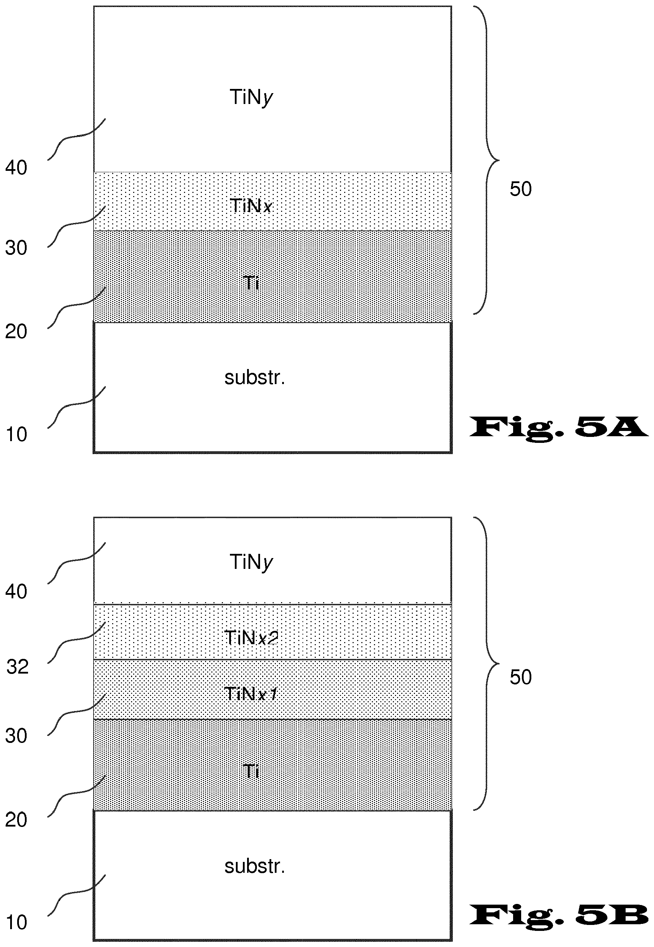

Figur 5A zeigt einen schematischen Querschnitt durch eine typische Schichtenfolge einer erfindungsgemäßen dreischichtigen Titanelektrode (50), die auf einem Substrat (10) abgeschieden die Grundkonfiguration eines erfindungsgemäßen Messmoduls bildet: Eine Ti-Schicht (20) ist direkt auf dem Substrat (10) abgeschieden, darauf eine titanreiche TiN*x*-Zwischenschicht (30), darauf eine titanarme TiN*y*-Deckschicht (40).
Figur 5B zeigt einen schematischen Querschnitt durch eine typische Schichtenfolge einer erfindungsgemäßen mehrschichtigen Titanelektrode (50) mit mehr als einer TiN*x*-Zwischenschicht: Eine Ti-Schicht (20) ist direkt auf dem Substrat (10) abgeschieden, darauf eine erste titanreiche TiN*xₙ*-Zwischenschicht (30), darauf mindestens eine weitere titanreiche TiN*x*_{*n*+1}-Zwischenschicht (32), die aber titanärmer ist, als die TiN*xₙ*-Zwisehenschicht, auf die sie abgeschieden ist, schließlich darauf eine titanarme TiN*y*-Deckschicht (40).

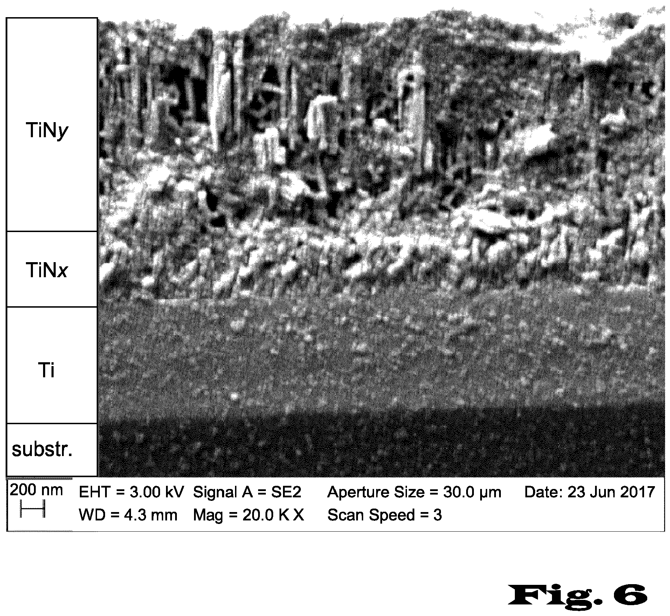

Figur 6 zeigt eine REM-Aufnahme eines Querschnitts durch eine erfindungsgemäße dreischichtige Titanelektrode (50): eine Schicht aus elementarem Titan ist direkt auf dem Substrat abgeschieden, darauf eine titanreiche TiN*x*-Zwischenschicht mit dichter Struktur, darauf eine titanarme TiN*y*-Deckschicht mit offener Struktur.

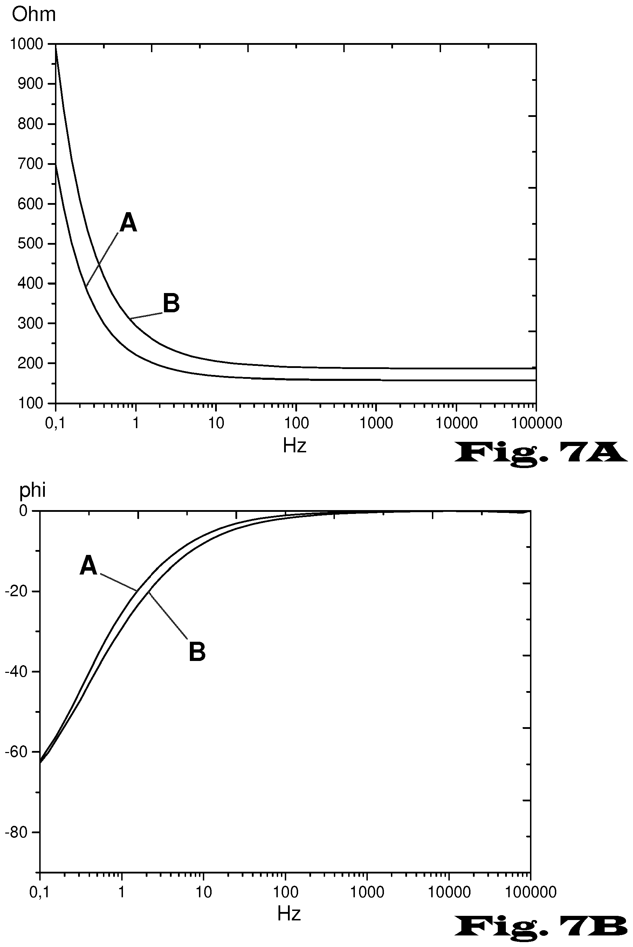

Figuren 7A und 7B zeigen Bode-Diagramme des Impedanzverlaufs realer dünner Titanelektroden: A= erfindungsgemäße dreischichtige Titanelektrode, B= einfache Ti/TiN-Elektrode. Figur 7A zeigt den Amplitudenverlauf (ohmscher Anteil), Figur 7B zeigt den Phasenverlauf.

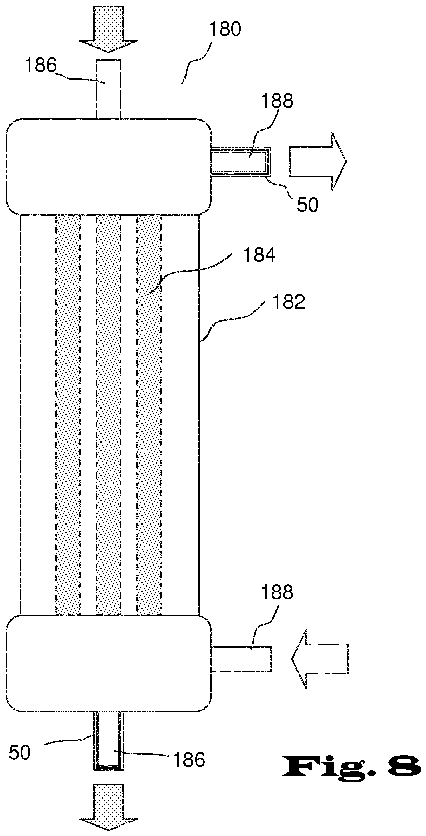

Figur 8 zeigt einen schematischen Querschnitt durch eine andere erfindungsgemäße Messanordnung in Form eines herkömmlichen Membranreaktors oder Dialysators mit einem Gehäuse (182) und darin angeordneten semipermeablen Hohlmembranen (184). Das Innere der Hohlmembranen (184) ist über Zu- und Abläufe (186) perfundierbar. Davon getrennt ist über Dialysat-Zu- und Abläufe (188) für der die Hohlmembranen (184) umgebende Raum perfundierbar. Erfindungsgemäß sind zumindest an einem Zu-oder Ablauf (186) und zumindest einem Dialysat-Zu- oder Ablauf des (188) jeweils eine erfindungsgemäße dreischichtige Titanelektrode (50) ausgebildet, um eine elektrische Messung der Impedanz über die Hohlmembranen (184) zu ermöglichen. Eine Besiedelung oder Verstopfung der Membran lässt sich so von extern durch laufende elektrische Messungen detektieren und quantifizieren.

### Beispiel 1: PVD-Prozess zur Herstellung von Ti-TiN Elektrodenschichten

Das thermoplastische Substrat, worauf die Titanelektrode erzeugt werden soll, wird in eine PVD-Beschichtungskammer eingesetzt, und in der Kammer wird über Vakuumpumpen (Vorpumpe und Turbopumpe) nach etwa 14 bis 18 Stunden ein Vakuum mit einem Druck von 1x10⁻⁶ mbar oder niedriger erreicht.

Beschichtungsvorbereitung 1: Die Kammer wird 5 min vor Beschichtungsbeginn mit einem Fluss von 274 sccm Argon geflutet, wobei der Gasfluss durch Massflow-Controller kontrolliert und geregelt wird.

Beschichtung 1: Titan-Trägerschicht: Beschichtung wird mit 500 W RF-Generatorleistung bei 274 sccm Argon-Fluss gestartet. Wenn Plasma zündet, wird direkt auf 100 sccm Argonfluss heruntergeregelt. Die Einstellung des reinen Argonflusses findet innerhalb weniger Sekunden nach dem Zünden statt. Ein 274 sccm Fluss wird nur benötigt, um die Plasmazündung zu erleichtern. Die Beschichtungsdauer beträgt 5 bis 30 Minuten, abhängig von der thermischen Belastbarkeit des Substratmaterials. Richtwerte sind hier 5 min bei Polystyrol, 15 min bei Polyamid, 30 min bei Edelstahlsubstraten

Zwischen der Ti-Beschichtung und nachfolgender TiNx-Beschichtung werden die jetzt mit Titan beschichteten Substrate bei einem Argon-Gasfluss von 100 sccm in der evakuierten Beschichtungskammer belassen. Sie kühlen unter diesen Bedingungen 1 bis 2 Stunden ab.

Beschichtungsvorbereitung 2: Argon-Fluss wird 5 min vor Beschichtungsbeginn von 100 sccm auf 274 sccm erhöht. Zusätzlich wird zeitgleich ein Stickstoff-Fluss von 2 sccm zugemischt, um das Prozessgas zu erzeugen

Beschichtung 2: TiNx-Zwischenschicht: Die Beschichtung wird mit 800 W RF-Generatorleistung bei 274 sccm Argon- und 2 sccm N2-Fluss gestartet. Wenn Plasma zündet wird direkt auf 180 sccm Argon-Fluss heruntergeregelt, um das eigentliche Prozessgas zu erzeugen. (Die Einstellung des reinen Argon-Flusses findet innerhalb weniger Sekunden nach dem Zünden statt. Der 274 sccm Fluss wird nur benötigt, um die Plasmazündung zu erleichtern.)

Die Beschichtungsdauer für die titanreiche TiN*x*-Schicht beträgt etwa 2 Minuten, und kann in Einzelfällen bei thermisch stabilen Substraten (z.B. Edelstahl) auf 3 Minuten erhöht werden.

Soll eine Titanelektrode mit mehreren titanreichen Zwischenschichten erzeugt werden, kann zur Abscheidung die Generatorleistung je nach gewünschtem Titananteil in kleineren Stufen gesenkt werden. Bei jeder Senkung der Generatorleistung wird eine Zwischenschicht mit geringerem Titangehalt als die vorhergehende Schicht abgeschieden. Gegebenenfalls kann es erforderlich sein, den erhöhten Argon-Fluss zu Beginn der Abscheidung zu erhöhen oder die Argon-Fluss Erhöhung über die gesamte Dauer der Abscheidung zumindest der TiN*x-*Zwischenschichten beizubehalten.

Beschichtung 3: TiN*y*-Deckschicht: Nach 2 bzw. 3 Minuten Beschichtung mit der titanreichen TiNx-Schicht bei 800W wird die Generatorleistung auf 500 W heruntergeregelt. Die Prozessgasmischung bleibt unverändert (180 sccm Argon- und 2 sccm N2-Fluss). Der Zerstäubungsprozess wird nicht gestoppt. Nun kann, auf die deponierte titanreiche TiNx-Schicht mit einer titanärmeren TiNy-Schicht beschichtet werden. Die Beschichtungsdauer hängt von den gewünschten elektrischen Eigenschaften der Elektrode (notwendige Schichtdicke) und von der thermischen Stabilität des Substrats ab. Richtwerte sind hier: Polystyrol: 5 bis 15 min, Polyamid: 30 min, Edelstahl: 90 min bis zu 150 min. Nach dieser Zeit wird der Generator abgeschaltet, um die Beschichtung zu stoppen.

Abschluss des Beschichtungsprozesses: Nach Abschalten des Generators bleiben die beschichteten Substrate abhängig von der vorhergehenden Beschichtungszeit bei laufenden Pumpen in der Prozessgas-Atmosphäre. Die Abkühlzeit entspricht halber Beschichtungszeit. Nach dieser ersten Abkühlphase in der Prozessgasmischung wird, der Stickstoffzufluss unterbrochen und der Argon-Fluss auf 100 sccm geregelt. Dieser Zustand wird wiederum für die halbe Beschichtungszeit beibehalten. Im Anschluss an diese zweite Abkühlphase wird der Argon-Fluss unterbrochen, das restliche Argon abgepumpt und anschließend die Vakuumpumpen gestoppt. Nach Auslaufen der Turbopumpe wird nochmals 5 bis 10 min gewartet, bevor die Kammer geöffnet wird und die beschichteten Substrate herausgenommen werden.

Ergebnisse: Die REM-Aufnahme in Figur 5 zeigt den Aufbau der Titanelektrode auf dem Substrat. Die Dicke der Beschichtung kann je nach Art und Temperaturverträglichkeit des Substrats bevorzugt zwischen 200 und 450 nm liegen. Für temperaturempfindlichere Substrate kann eine dünnere titanreiche Zwischenschicht (TiN*x*) deponiert werden, um mehr "Wärmebudget" für die anschließende titanärmere Deckschicht (TiN*y*) zu reservieren. Für metallische Substrate kann die titanreiche Zwischenschicht (TiN*x*) auch dicker, besonders bis zu 450 nm, ausgeführt werden.

Die TiN*x*-Zwischenschicht besitzt eine höhere Dichte, als die titanärmere TiN*y-*Deckschicht. Dies bedingt eine gute Leitfähigkeit der Zwischenschicht. Die offenere Struktur der titanärmeren TiN*y*-Deckschicht bedingt deren verbessertes Impedanzverhalten.

### Beispiel 2: Impedanzverhalten der Titanelektrode

Auf polymeren, thermisch sensitiven Trägern (Polystyrol) werden sehr dünne Titanelektroden abgeschieden. Zum einen werden Ti/TiN-Elektroden mit herkömmlichem zweischichtigem Aufbau hergestellt, zum anderen erfindungsgemäße dreischichtige Titanelektroden aus Ti/TiN*x*/TiN*y* gemäß Beispiel 1 hergestellt. In beiden Fällen werden die Beschichtungsparameter so gewählt, dass nur dünne Schichten erzeugt werden, um die thermisch sensitiven Träger nicht zu schädigen.

Das Impedanzverhalten in Widerstand (Ohmsche Komponente) und Phasenwinkel (Bode-Diagramm) ist bei den erfindungsgemäßen dreischichtigen Titanelektroden, vor allem in dem für die impedanzspektroskopischen TEER-Messungen von biologischem Gewebe relevanten Frequenzbereich signifikant verbessert: der Serienwiderstand der erfindungsgemäßen Elektrode (A) ist kleiner und zeigt eine geringere Frequenzabhängigkeit (Figur 7A), der Phasenverlauf ist flacher, die Phasenverschiebung kleiner (Figur 7B).

## Patentansprüche

1. Titanelektrode (50) für die Impedanzmessung in wässrigen biologischen Medien, enthaltend auf einem Substrat (10):
- eine Ti-Trägerschicht (20), bestehend aus elementarem Titan (Ti),
- eine oder mehrere titanreiche Titannitrid (TiN*ₓ*)-Zwischenschichten (30,32), bestehend aus TiN*ₓ*, mit x kleiner als 1 oder x gleich 1, welche auf der Ti-Trägerschicht aufliegt, und
- eine titanarme Titannitrid (TiN*_{y}*)-Deckschicht (40), bestehend aus TiN*_{y}* mit *y* größer als 1, welche auf der titanreichen TiN*ₓ*-Zwischenschicht aufliegt.

2. Titanelektrode (50) nach Anspruch 1, wobei die Dicke der TiN*_{y}*-Deckschicht (40) das 0,5- bis 10-Fache, bevorzugt das 1,5- bis 4-Fache, der Dicke der TiN*ₓ*-Zwischenschicht (30) beträgt.

3. Titanelektrode (50) nach Anspruch 2, wobei die drei Elektrodenschichten (20, 30, 40) ein Dickenverhältnis von 1-5 / 1 / 0,5-10, bezogen auf Ti-Trägerschicht (20) / TiN*ₓ*-Zwischenschicht (30) / TiN*_{y}*-Deckschicht (40), besitzen.

4. Titanelektrode (50) nach einem der vorstehenden Ansprüche, wobei jede TiN*ₓ*-Zwischenschicht (30,32) eine Dicke von 100 bis 500 nm, bevorzugt von 150 bis 250 nm aufweist.

5. Titanelektrode (50) nach einem der vorstehenden Ansprüche, wobei die TiN*ₓ*-Zwischenschicht (30,32) aus TiN*ₓ* besteht, mit x von 0,3 bis 1,0.

6. Titanelektrode (50) nach einem der vorstehenden Ansprüche, wobei die TiN*_{y}*-Deckschicht (40) aus TiN*_{y}* besteht, mit *y* von 1,1 bis 1,5

7. Messmodul (150, 160, 180) für die Impedanzmessung in wässrigen biologischen Medien, enthaltend:
- ein polymeres Substrat (10) zur Führung von flüssigem Medium und
- eine unmittelbar auf dem Substrat deponierte mehrschichtige Titanelektrode (50) nach einem der vorstehenden Ansprüche.

8. Messmodul nach Anspruch 7, wobei das polymere Substrat (10) ausgewählt ist aus der Gruppe der Thermoplaste, bevorzugt bestehend aus: Polystyrol (PS), Polyamid (PA), Acrylnitril-Butadien-Styrol (ABS), Polylactat (PLA), Polycarbonat (PC), Polyethylenterephthalat (PET), Polyethylen (PE), Polycaprolacton (PCT), Polypropylen (PP), Polyetheretherketon (PEEK), Polyimid (PI), Polyurethan (PU) und Polyvinylchlorid (PVC) sowie Kombinationen, Mischungen und Co-Polymeren davon.

9. Messmodul nach Anspruch 7 oder 8, ausgewählt aus: Zellkulturgefäßen, Multiwellplatten, Membranreaktoren, Hohlfasermodulen, Schlauchanschlussstücken.

10. Verfahren zur Herstellung einer Titanelektrode (50) für die Impedanzmessung mittels reaktiver Kathodenzerstäubung in einem Prozessgas, enthaltend Argon (Ar) und Stickstoff (N₂), enthaltend die Schritte:
b) Sputtern von Titan auf einem Substrat aus elementarem Titan der Elektrode bei erhöhter Generatorleistung, um mindestens eine erste Titannitridzwischenschicht (30), bestehend aus titanreichem TiN*x* mit *x* kleiner als 1 oder x gleich 1, auf einer Titanschicht (20) zu deponieren,
c) umittelbar anschließend: Reduzieren der Generatorleistung und
d) Sputtern von Titan auf das Substrat bei der verminderten Generatorleistung um eine Titannitriddeckschicht (40), bestehend aus titanarmem TiN*y* mit *y* größer als 1 auf der in Schritt b) gebildeten Titannitridzwischenschicht (30, 32) zu deponieren.

11. Verfahren nach Anspruch 10, wobei die Schritte b) und c) mehrfach durchlaufen werden, um mindestens eine weitere Titannitridzwischenschicht (32), bestehend aus dem titanreichem TiN*x*, auf einer ersten Titannitridzwischenschicht (30) zu deponieren, wobei in den Schritten c) bei jedem Durchlauf die Generatorleistung jeweils gegenüber dem vorhergehenden Durchlaufs reduziert wird, um eine titanärmere weitere Titannitridzwischenschicht (32) auf einer der jeweils zuvor abgeschiedenen titanreicheren Titannitridzwischenschicht (30) zu bilden.

12. Verfahren nach Anspruch 10 oder 11, wobei die erhöhte Generatorleistung in Schritt b) das 1,2- bis 2,4-Fache der Generatorleistung in Schritt d) beträgt.

13. Verfahren nach einem der Ansprüche 10 bis 12, wobei während der Durchführung Schritte b) bis d) die Zusammensetzung des Prozessgases (Ar/N₂), bezogen auf den N₂-Partialdruck, konstant gehalten wird.

14. Verfahren nach einem der Ansprüche 10 bis 12, weiter enthaltend einen dem Schritt b) vorangehenden Schritt:
a) Deponieren einer Titanschicht (20) auf einem Elektrodensubstrat (10).

15. Verfahren zur Herstellung eines Messmoduls für die Impedanzmessung in wässrigen biologischen Medien, enthaltend die Schritte:
- Bereitstellen des medienführenden Moduls als Substrat der Elektrode und
- Abscheiden der Titanelektrode auf diesem Substrat mit dem Verfahren nach Anspruch 13 oder 14.

## Claims

1. A titanium electrode (50) for the impedance measurement in aqueous biological media, containing on a substrate (10):
- a Ti carrier layer (20), consisting of elemental titanium (Ti),
- one or several titanium-rich titanium nitride (TiN*ₓ*) intermediate layers (30,32), consisting of TiN*ₓ*, with *x* smaller than 1 or *x* equal to 1, which rests on the Ti carrier layer, and
- a titanium-poor titanium nitride (TiN*_{y}*) cover layer (40), consisting of TiN*_{y}* with y greater than 1, which rests on the titanium-rich TiN*ₓ* intermediate layer.

2. The titanium electrode (50) according to claim 1, wherein the thickness of the TiN*_{y}* cover layer (40) is 0.5 to 10 times, preferably 1.5 to 4 times, the thickness of the TiN*ₓ* intermediate layer (30).

3. The titanium electrode (50) according to claim 2, wherein the three electrode layers (20, 30, 40) have a thickness ratio of 1-5 / 1 / 0.5-10, based on Ti carrier layer (20) / TiN*ₓ* intermediate layer (30) / TiN*_{y}* cover layer (40).

4. The titanium electrode (50) according to any one of the preceding claims, wherein each TiN*ₓ* intermediate layer (30,32) has a thickness from 100 to 500 nm, preferably from 150 to 250 nm.

5. The titanium electrode (50) according to any one of the preceding claims, wherein the TiN*ₓ* intermediate layer (30,32) consists of TiN*ₓ*, with x from 0.3 to 1.0.

6. The titanium electrode (50) according to any one of the preceding claims, wherein the TiN*_{y}* cover layer (40) consists of TiN*_{y}*, with *y* from 1.1 to 1.5.

7. A measuring module (150, 160, 180) for the impedance measurement in aqueous biological media, containing:
- a polymeric substrate (10) for guiding liquid medium, and
- a multilayer titanium electrode (50) according to any one of the preceding claims, which is deposited immediately on the substrate.

8. The measuring module according to claim 7, wherein the polymeric substrate (10) is selected from the group of the thermoplastics, preferably consisting of:
polystyrene (PS), polyamide (PA), acrylonitrile butadiene styrene (ABS), polylactic acid (PLA), polycarbonate (PC), polyethylene terephthalate (PET), polyethylene (PE), polycaprolactone (PCT), polypropylene (PP), polyetheretherketone (PEEK), polyimide (PI), polyurethane (PU), and polyvinylchloride (PVC) as well as combinations, mixtures, and co-polymers thereof.

9. The measuring module according to claim 7 or 8, selected from: cell culture vessels, multi-well plates, membrane reactors, hollow fiber modules, hose connecting pieces.

10. A method for producing a titanium electrode (50) for the impedance measurement by means of reactive cathode sputtering in a process gas containing argon (Ar) and nitrogen (N₂), including the steps:
b) sputtering titanium onto an elemental titanium substrate of the electrode at increased generator power, in order to deposit at least a first titanium nitride intermediate layer (30), consisting of titanium-rich TiN*x* with *x* smaller than 1 or x equal to 1, on a titanium layer (20),
c) immediately following: reducing the generator power, and
d) sputtering titanium onto the substrate at the reduced generator power in order to deposit a titanium nitride cover layer (40), consisting of titanium-poor TiN*y* with y greater than 1 on the titanium nitride intermediate layer (30,32) formed in step b).

11. The method according to claim 10, wherein the steps b) and c) are passed through several times, in order to deposit at least one further titanium nitride intermediate layer (32), consisting of the titanium-rich TiN*x*, on a first titanium nitride intermediate layer (30), wherein in steps c) for each pass-through the generator power is in each case reduced as compared to the previous pass-through, in order to form a titanium-poorer further titanium nitride intermediate layer (32) on one of the respective titanium-richer titanium nitride intermediate layer (30), which had in each case been deposited beforehand.

12. The method according to claim 10 or 11, wherein the increased generator power in step b) is 1.2 to 2.4 times the generator power in step d).

13. The method according to any one of claims 10 to 12, wherein the composition of the process gas (Ar/N₂), based on the N₂ partial pressure, is held constant while performing steps b) to d).

14. The method according to any one of claims 10 to 12, further including a step preceding step b):
a) depositing a titanium layer (20) on an electrode substrate (10).

15. A method for producing a measuring module for the impedance measurement in aqueous biological media, including the steps:
- providing the media-guiding module as substrate of the electrode, and
- depositing the titanium electrode on this substrate by means of the method according to claim 13 or 14.

## Revendications

1. Une électrode en titane (50) pour la mesure d'impédance dans des milieux biologiques aqueux, contenant sur un substrat (10) :
- une couche support de Ti (20), constituée de titane élémentaire (Ti),
- une ou plusieurs couches intermédiaires (30, 32) en nitrure de titane (TiNₓ) riches en titane, constituées de TiNₓ, avec x inférieur à 1 ou x égal à 1, qui repose sur la couche de support en Ti, et
- une couche de couverture (40) en nitrure de titane pauvre en titane (TiN_{y}), constituée de TiN_{y} avec y supérieur à 1, qui repose sur la couche intermédiaire en TiNₓ riche en titane.

2. L'électrode en titane (50) selon la revendication 1, dans laquelle l'épaisseur de la couche de couverture en TiN_{y} (40) est de 0,5 à 10 fois, de préférence de 1,5 à 4 fois, l'épaisseur de la couche intermédiaire en TiNₓ (30).

3. L'électrode en titane (50) selon la revendication 2, dans laquelle les trois couches d'électrode (20, 30, 40) ont un rapport d'épaisseur de 1-5/1 / 0,5-10, sur la base de la couche support en Ti (20) / couche intermédiaire en TiNₓ (30) / couche de couverture en TiN_{y} (40).

4. L'électrode en titane (50) selon l'une quelconque des revendications précédentes, dans laquelle chaque couche intermédiaire en TiNₓ (30, 32) a une épaisseur de 100 à 500 nm, de préférence de 150 à 250 nm.

5. L'électrode en titane (50) selon l'une quelconque des revendications précédentes, dans laquelle la couche intermédiaire en TiNₓ (30, 32) est constituée de TiNₓ, avec x de 0,3 à 1,0.

6. L'électrode en titane (50) selon l'une quelconque des revendications précédentes, dans laquelle la couche de couverture (40) en TiN_{y} est constituée de TiN_{y}, avec y de 1,1 à 1,5.

7. Un Module de mesure (150, 160, 180) pour la mesure d'impédance dans des milieux biologiques aqueux, contenant :
- un substrat polymèrique (10) pour le guidage du milieu liquide, et
- une électrode multicouche en titane (50) selon l'une quelconque des revendications précédentes, qui est déposée immédiatement sur le substrat.

8. Le module de mesure selon la revendication 7, dans lequel le substrat polymèrique (10) est choisi dans le groupe des thermoplastiques, de préférence constitué par : le polystyrène (PS), le polyamide (PA), l'acrylonitrile butadiène styrène (ABS), l'acide polylactique (PLA), le polycarbonate (PC), le polyéthylène téréphtalate (PET), le polyéthylène (PE), le polycaprolactone (PCT), le polypropylène (PP), le polyétheréthercétone (PEEK), le polyimide (PI), le polyuréthane (PU) et le polychlorure de vinyle (PVC) ainsi que leurs combinaisons, mélanges et copolymères.

9. Le module de mesure selon la revendication 7 ou 8, choisi parmi : les récipients de culture cellulaire, les plaques multi-puits, les réacteurs à membrane, les modules à fibres creuses, les pièces de raccordement de tuyaux.

10. Un procédé de fabrication d'une électrode en titane (50) pour la mesure de l'impédance au moyen d'une pulvérisation cathodique réactive dans un gaz de traitement contenant de l'argon (Ar) et de l'azote (N₂), comprenant les étapes suivantes :
b) pulvérisation de titane sur un substrat de titane élémentaire de l'électrode à une puissance de générateur accrue, afin de déposer au moins une première couche intermédiaire de nitrure de titane (30), constituée de TiNₓ riche en titane avec x inférieur à 1 ou x égal à 1, sur une couche de titane (20),
c) immédiatement après : réduire la puissance du générateur, et
d) la pulvérisation de titane sur le substrat à la puissance réduite du générateur afin de déposer une couche de couverture de nitrure de titane (40), constituée de TiN_{y} pauvre en titane avec y supérieur à 1 sur la couche intermédiaire de nitrure de titane (30,32) formée à l'étape b).

11. Le procédé selon la revendication 10, dans lequel les étapes b) et c) sont passées plusieurs fois, afin de déposer au moins une autre couche intermédiaire en nitrure de titane (32), constituée du TiNx riche en titane, sur une première couche intermédiaire en nitrure de titane (30), dans lequel, dans les étapes c), pour chaque passage, la puissance du générateur est à chaque fois réduite par rapport au passage précédent, afin de former une autre couche intermédiaire en nitrure de titane (32) plus pauvre en titane sur l'une des couches intermédiaires en nitrure de titane (30) respectives plus riches en titane, qui avaient à chaque fois été déposées auparavant.

12. Le procédé selon la revendication 10 ou 11, dans lequel la puissance du générateur augmentée à l'étape b) est de 1,2 à 2,4 fois la puissance du générateur à l'étape d).

13. Le procédé selon l'une quelconque des revendications 10 à 12, dans lequel la composition du gaz de traitement (Ar/N₂), basée sur la pression partielle de N₂, est maintenue constante pendant la réalisation des étapes b) à d).

14. Le procédé selon l'une quelconque des revendications 10 à 12, comprenant en outre une étape précédant l'étape b) :
a) dépôt d'une couche de titane (20) sur un substrat d'électrode (10).

15. Un procédé de fabrication d'un module de mesure de l'impédance dans des milieux biologiques aqueux, comprenant les étapes suivantes :
- fournir le module de guidage du milieu comme substrat de l'électrode, et
- déposer l'électrode en titane sur ce substrat au moyen du procédé selon la revendication 13 ou 14.
